# EUROPEAN PATENT APPLICATION

(11) **EP 4 628 039 A1**
(43) Date of publication of application: **08.10.2025**
(21) Application number: 23896731.9
(22) Date of filing: 27.11.2023
(51) Int. Cl.: A61B 18/18, A61B 18/20, H05K 7/20, H05K 5/02, A61N 5/06

(54) **HAIR REMOVAL DEVICE**

(30) Priority: 02.12.2022 CN 202223240301 U
(71) Applicant: Godox Photo Equipment Co., Ltd., Shenzhen, Guangdong 518000 (CN)
(72) Inventor: ZENG, Weijun, Shenzhen, Guangdong 518000 (CN)
(74) Representative: Studio Torta S.p.A.
(86) International application number: PCT/CN2023/134344
(87) International publication number: WO 2024/114580

(57) **Abstract**

The present application provides a hair removal device, pertaining to the technical field of beauty instruments. The hair removal device comprises a housing, a pulse light source, an air duct, and a fan. The housing is hollow inside and provided with a light outlet, an air inlet, and an air outlet; the pulse light source is arranged in the housing, and pulse light emitted by the pulse light source is emitted from the light outlet; the air duct is arranged in the housing and is in communication with the air inlet and the air outlet; the air duct passes through both sides of the pulse light source; the fan is arranged in the air duct and used for generating gas flow, so that the airflow enters through the air inlet and flows out through the air outlet by means of the air duct. Part of the air duct is arranged on both sides of the pulse light source, so that the technical problem of poor heat dissipation effect of traditional hair removal devices, which restricts the improvement of hair removal efficiency of the devices, is solved.

## Description

### CROSS-REFERENCE TO RELATED APPLICATIONS

This application claims priority to a Chinese patent application No. 202223240301.0, titled "Hair Removal Device", filed on December 2, 2022, the whole disclosure of which is incorporated into this application by reference.

### TECHNICAL FIELD

The present application relates to the technical field of beauty apparatus, particularly to a hair removal device.

### BACKGROUND

The traditional photon hair removal device removes hair through the use of pyrolysis principle of a preset wavelength of intense pulse light source, in order to improve the efficiency of hair removal, and the traditional hair removal device aims to increase the number of light flashes, but frequent high-power flash will inevitably generate a large amount of heat, too much heat will lead to the device that cannot be used normally or even damage to the device. Due to the poor heat dissipation of the current hair removal device, in order to ensure the safety and stability of the hair removal device, the hair removal device on the current market needs only reduce the output power, which greatly reduces the efficiency of the hair removal device, resulting in extension of the user's use of time and reduction of the user's experience.

### SUMMARY

An object of the present application is to solve the technical problem that the traditional hair removal device has poor heat dissipation effect, which limits the efficiency of hair removal of the device.

In order to solve the above technical problems, the present application provides a hair removal device, including: a housing, hollow inside, wherein the housing is provided with a light outlet, an air inlet and an air outlet; a pulse light source, provided inside the housing, wherein pulse light emitted by the pulse light source is emitted from the light outlet; an air duct, provided inside the housing and in communication with the air inlet and the air outlet, wherein the air duct passes through two sides of the pulse light source, respectively; and a fan, provided inside the air duct for generating an airflow, to make the airflow enter from the air inlet and pass through the air duct and exit from the air outlet.

In one embodiment, the air duct includes an air inlet duct, a working duct and an air outlet duct communicated sequentially, the working duct includes a first working duct and a second working duct, the first working duct is located on a side of the pulse light source where the pulse light is emitted from, and the second working duct is located a side of the pulse light source away from the side of the pulse light source where the pulse light is emitted from.

In one embodiment, the hair removal device further includes a reflective cover and a light filter, and an opening at an end of the reflective cover and the hollow inside of the reflective cover form a reflective cavity, wherein the pulse light source is provided in the reflective cavity, and the light filter is provided at an end of the reflective cover from which the pulse light is emitted for sealing the reflective cover.

In one embodiment, two sidewalls of the reflective cover are opened with vents opposite to each other, each vent is configured to communicate the air inlet duct with the air outlet duct to form the first working duct for the airflow flowing along the first working duct from a front side of the pulse light source.

In one embodiment, the hair removal device further includes a first bracket, the first bracket is provided with a mounting groove, and an open end of the mounting groove points to a direction where the pulse light is emitted.

In one embodiment, the reflective cover is provided in the mounting groove, a gap is provided between an outer wall of the reflective cover and a bottom wall of the mounting groove for communicating the air inlet duct with the air outlet duct to form the second working duct for the airflow flowing along the second working duct from a rear side of the pulse light source.

In one embodiment, the hair removal device further includes a second bracket, the first bracket is provided with a first accommodation cavity and the second bracket is provided with a second accommodation cavity, wherein the second bracket is snapped to the first bracket, to make the first accommodation cavity and the second accommodation cavity combine to form a mounting cavity; and a side of the first bracket facing the air outlet is opened with a through hole, the through hole is configured to communicate the mounting cavity with the air outlet, a side of the mounting cavity away from the through hole is provided with an opening, and the opening is configured to communicate with the working duct to form the air outlet duct.

In one embodiment, the hair removal device further includes a light gathering assembly, and the light gathering assembly is provided on a side of the light filter away from the pulse light source, wherein a space is formed between the first bracket and the light gathering assembly, and is configured to communicate the air inlet with the working duct to form the air inlet duct.

In one embodiment, the light gathering assembly includes a light gathering frame, a fixing frame and a light outlet cap; the light outlet cap is connected to the housing, and the light gathering frame is provided within the fixing frame, the light filter, the light gathering frame and the light outlet cap are arranged sequentially along a direction where the pulse light is emitted.

In one embodiment, a middle of the light outlet cap is opened with a light outlet for the pulse light to be emitted.

In one embodiment, the fixing frame is provided at an inner end of the light outlet cap, the light gathering frame is provided inside the fixing frame, and the light filter is provided at an end of the fixing frame away from the light outlet cap.

In one embodiment, the fan is provided inside the mounting cavity.

In one embodiment, the air inlet and the air outlet are provided spaced apart on the housing, and an area of the air outlet is larger than an area of the air inlet.

In one embodiment, the air inlet and the air outlet are provided on the same side of the housing.

In one embodiment, the housing is further provided with a display screen and a function button, and the display screen and the function button are located on a side of the housing away from the air inlet and the air outlet.

In one embodiment, the housing includes a first housing and a second housing, and the first housing is detachably connected to the second housing.

As can be seen from the above technical solutions, the beneficial effects of the present application are:
The present application provides a hair removal device, which includes a housing with a hollow inside, the housing is provided with a light outlet, an air inlet and an air outlet. A pulse light source is provided inside the housing, and the pulse light source emits the pulse light from the light outlet to remove hair from the skin. An air duct is provided inside the housing, and the air duct is in communication with the air inlet and the air outlet, and the air duct passes from two sides of the pulse light source. A fan is provided in the air duct, and the fan generates an airflow, so that the airflow enters from the air inlet and passes through the air duct and then flows out from the air outlet. A portion of the air duct is provided on two sides of the pulse light source, which can increase the heat dissipation area of the pulse light source, and the airflow can quickly take away the heat generated by the lamp assembly, greatly improving the heat dissipation effect of the hair removal device, so that the hair removal device can use a higher output power of the pulse light source, to improve the efficiency of the hair removal, thereby reducing the user's use time and improving the user's experience.

### BRIEF DESCRIPTION OF THE DRAWINGS

FIG. 1 is a front view structure of the beauty device.
FIG. 2 is a rear view structure of the beauty device.
FIG. 3 is a right view structure of the beauty device.
FIG. 4 is a cross-sectional structure of the beauty device at line A-A in FIG. 1.
FIG. 5 is a partially enlarged view of the cross-sectional structure of the beauty device at line A-A in FIG. 4.
FIG. 6 is a view of the disassembled structure of the beauty device.
FIG. 7 is an enlarged view of a part of the beauty device in FIG. 6.

### Number references:

100, a hair removal device; 10, a housing; 101, a mounting cavity; 11, a first housing; 111, an air inlet; 112, an air outlet; 12, a second housing; 20, an air duct; 21, an air inlet duct; 22, a working duct; 221, a first working duct; 222, a second working duct; 23, an air outlet duct; 30, a pulse light source; 31, a reflective cover ; 311, a vent; 32, a filter; 40, a light gathering assembly; 41, a light gathering frame; 42, a fixing bracket; 43, a light outlet cap; 431, a light outlet; 50, a first bracket; 51, a front baffle; 52, a main portion; 521, a mounting groove; 522, a through hole; 53, first accommodation cavity; 60, a second bracket; 61, a second accommodation cavity; 70, a fan; 80, a display screen; 90, a function button.

### DETAILED DESCRIPTION OF THE EMBODIMENTS

Embodiments embodying the features and advantages of the present application will be described in detail in the following description. It is to be understood that the present application is capable of various variations in different embodiments, none of which is outside the scope of the present application, and that the descriptions and drawings therein are intended to be illustrative in nature and are not intended to limit the present application.

In the description of the present application, it is to be understood that in the embodiments shown in the accompanying drawings, indications of orientation or positional relationships (such as up, down, left, right, forward and backward, etc.) are provided solely for the purpose of facilitating and simplifying the description of the present application, rather than indicating or implying that the device or element referred to must have a particular orientation, be constructed and operated in a particular orientation. These descriptions are appropriate when these elements are in the positions shown in the accompanying drawings. If the description of the position of these elements is changed, the indications of these orientations are changed accordingly.

Furthermore, the terms "first" and "second" are used only for descriptive purposes and are not to be understood as indicating or implying relative imopeningance or implicitly specifying the number of technical features indicated. Thus, a feature defined with "first", "second" may expressly or implicitly include one or more of the described features. In the description of the present application, "more than one" means two or more, unless otherwise expressly and specifically limited.

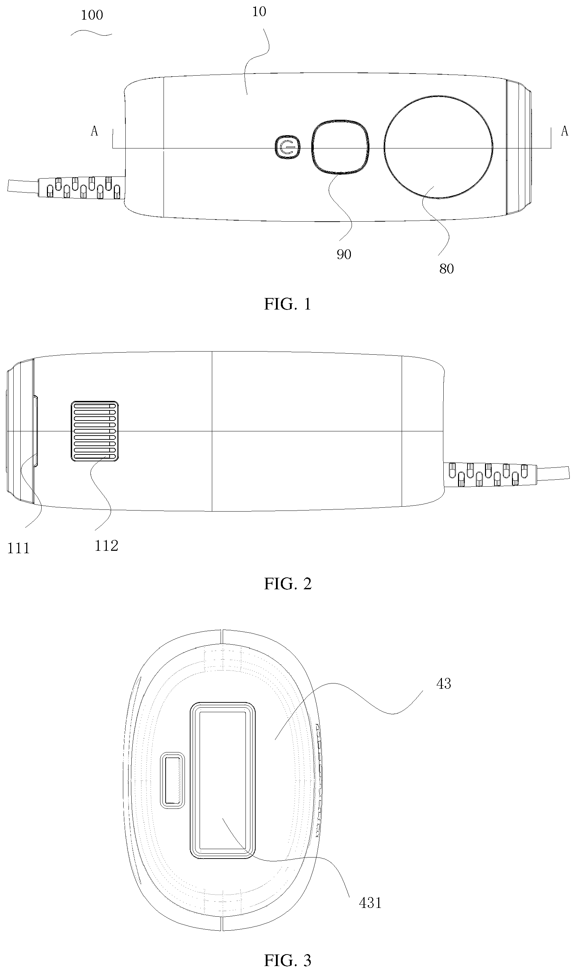

Referring to FIGS. 1 to 7, the present embodiment provides a hair removal device 100, which includes a housing 10, a pulse light source 30, an air duct 20, and a fan 70. The pulse light source 30 is provided in a hollow inside of the housing 10, and the pulse light source 30 emits pulse light, which is emitted from a light outlet 431 on the housing 10, so that the pulse light is emitted to the skin for hair removal.

The housing 10 is provided with an air inlet 111 and an air outlet 112, and the airflow can enter from the air inlet 111 and flow out from the air outlet 112. The air duct 20 is provided inside the housing 10 and is in communication with the air inlet 111 and the air outlet 112. The air duct 20 passes through two sides of the pulse light source 30, respectively, and the fan 70 is also provided inside the air duct 20 for generating the airflow, which enters from the air inlet 111, flows through the air duct 20 from two sides of the pulse light source 30, and then flows out from the air outlet 112. The air duct 20 is provided on two sides of the pulse light source 30, such that the heat dissipation area of the pulse light source 30 is increased, and the heat generated by the lamp assembly can be rapidly taken away, greatly improving the heat dissipation effect of the hair removal device 100.

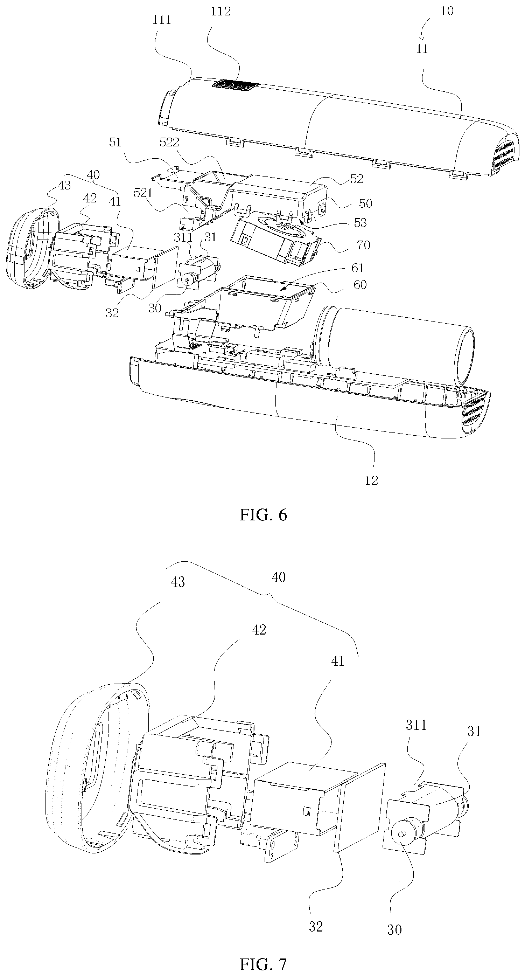

Referring to FIG. 6, the hair removal device 100 of the present embodiment further includes a first bracket 50 provided in the housing 10, a second bracket 60 snapped with the first bracket 50, a reflective cover 31 provided on the first bracket 50, a light filter 32 attached to the reflective cover 31, and a light gathering assembly 40 provided on a side of the light filter 32 away from the reflective cover 31.

The air duct 20 in this example is formed by the specific structures or relative mounting positions of the reflective cover 31, the light filter 32, the light gathering assembly 40, the first bracket 50 and the second bracket 60. It is to be understood that the air duct 20 may also be formed by the hollowing out of the internal structure of the housing 10, which is sufficient to satisfy that the air duct 20 passes through two sides of the pulse light source 30, respectively, so that the airflow rapidly takes away the heat emitted by the pulse light source 30.

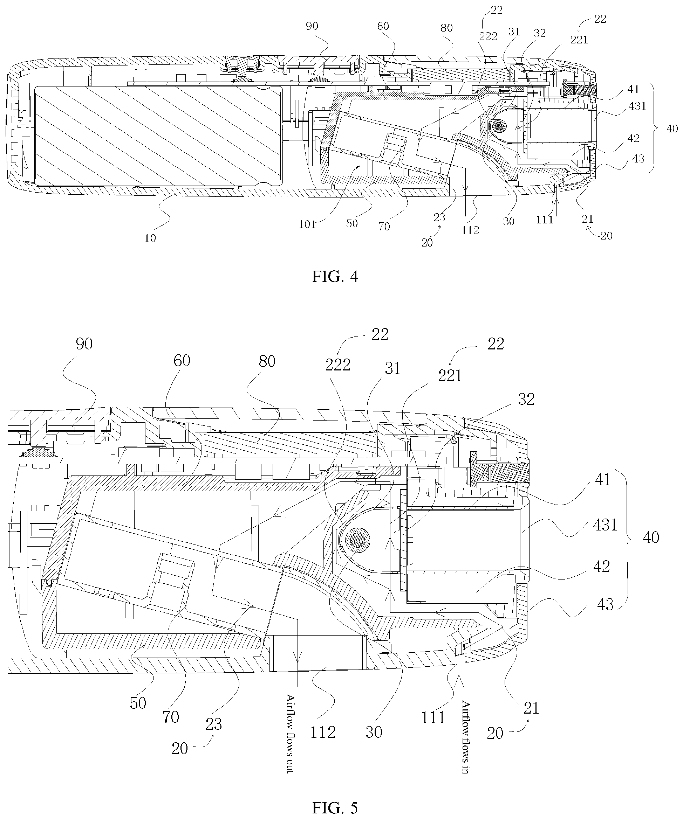

Referring to FIGS. 4 and 5, the air duct 20 in this embodiment includes an air inlet duct 21, a working duct 22, and an air outlet duct 23. The working duct 22 includes a first working duct 221 and a second working duct 222, and the first working duct 221 is located on a side of the pulse light source 30 where the pulse light is emitted from, and the second working duct 222 is located on the side of the pulse light source 30 away from the side of the pulse light source 30 where the pulse light is emitted from.

The working duct 22 defined in this embodiment is provided on two sides of the pulse light source 30 for directly taking away heat from the pulse light source 30. The air inlet duct 21 is provided between the working duct 22 and the air inlet 111. The air outlet duct 23 is provided between the working duct 22 and the air outlet 112.

Referring to FIGS. 5 and 6, the air inlet duct 21 is formed by a space between the light gathering assembly 40 and the first bracket 50.

Specifically, the light gathering assembly 40 includes a light gathering frame 41, a fixing frame 42, and a light out cap 43. The light out cap 43 is threadedly connected to the housing 10, and a light outlet 431 is opened in a middle of the light out cap 43 for the pulse light to be emitted. The fixing frame 42 is provided at an inner end of the light outlet cap 43 for fixing the light gathering frame 41 and the light filter 32. The light gathering frame 41 is provided inside the fixing frame 42, and the light filter 32 is provided at an end of the fixing frame 42 away from the light outlet cap 43. The light filter 32, the light gathering frame 41 and the light outlet cap 43 are sequentially arranged along a direction where the pulse light is emitted, so that the pulse light emitted from the pulse light source 30 passes through the light filter 32 and the light gathering frame 41 in turn, and then is emitted from the light outlet 431.

The first bracket 50 is provided at a rear side of the light gathering assembly 40 and a space is formed between the first bracket 50 and the light gathering assembly 40, the space communicates the air inlet 111 with the working duct 22 to form the air inlet duct 21.

Exemplarily, the first bracket 50 includes a front baffle 51 and a main body portion 52, the front baffle 51 is provided at a front end of the main body portion 52, and there is a gap between the front baffle 51 and the fixing bracket 42, and a gap between the front baffle 51 and the light outlet cap 43, so that the front baffle 51 together with the outer wall of the fixing bracket 42 and the inner wall of the light outlet cap 43 form the air inlet duct 21.

Referring to FIGS. 5 and 6, the air outlet duct 23 includes a first bracket 50 and a second bracket 60 snapped with each other.

Specifically, the first bracket 50 is provided with a first accommodation cavity 53, and the second bracket 60 is provided with a second accommodation cavity 61. The second bracket 60 is snapped together with the first bracket 50, so that the first accommodation cavity 53 and the second accommodation cavity 61 combine to form a mounting cavity 101. A through hole 522 is opened on the side of the first bracket 50 toward the air outlet 112, and the through hole 522 communicates the mounting cavity 101 with the air outlet 112. An opening is provided on a side of the mounting cavity 101 away from the through hole 522, which communicates the mounting cavity 101 with the working duct 22 to form an air outlet duct 23.

The mounting cavity 101 in this embodiment is formed by the first bracket 50 and the second bracket 60 snapping together and is directly communicated with the air outlet 112, so that the sidewall of the mounting cavity 101 isolates the air outlet duct 23 from the devices such as the battery, the motherboard, and the display 80 within the housing 10. The airflow, after passing through the pulse light source 30, enters the mounting cavity 101 through the opening of the mounting cavity 101 and then flows out directly through the air outlet 112, which prevents the airflow from flowing to other regions within the housing 10 and thermally affecting other electronic devices.

Referring to FIGS. 4 to 7, the first working duct 221 located at the front side of the pulse light source 30 is formed by a reflective cover 31 and a light filter 32.

Specifically, the reflective cover 31 is opened at one end and hollow inside to form a reflective cavity, and the pulse light source 30 is provided in the reflective cavity, which is configured to reflect the pulse light, so that the pulse light is emitted in one direction. A light filter 32 is provided at the end of the reflective cover 31 from which the pulse light is emitted and seals the reflective cover 31. The two sidewalls of the reflective cover 31 are relatively open with vents 311, which communicate the air inlet duct 21 with the air outlet duct 23 to form a first working duct 221, and the airflow can flow along the first working duct 221 from the front side of the pulse light source 30 to bring out the heat inside the reflective cover 31.

It is to be understood that the vent 311 may be a notch opened at the edge of the reflective cover 31, which together with the light filter 32 forms the vent 311. Of course, the vent 311 may also be a hole provided in the middle of the two sidewalls of the reflective cover 31, as long as it can communicate the space formed by the reflective cover 31 and the light filter 32 with the inlet air duct 21 and the outlet air duct 23.

Please continue to refer to FIGS. 5 and 6, the second working duct 222 located on a back side of the pulse light source 30 is formed by the reflective cover 31 and the mounting groove 521 of the first bracket 50.

Specifically, the first bracket 50 is provided with a mounting groove 521, and an open end of the mounting groove 521 points to a direction where the pulse light is emitted. The reflective cover 31 is provided in the mounting groove 521, and there is a gap between the back side of the reflective cover 31 and the bottom wall of the mounting groove 521, and the gap communicates the air inlet duct 21 with the air outlet duct 23 to form a second working duct 222. An airflow may flow along the second working duct 222 from the back side of the pulse light source 30 to take away heat in the back side of the reflective cover 31.

Referring to FIG. 6, the housing 10 includes a first housing 11 and a second housing 12. The first housing 11 and the second housing 12 are connected by a snap to form a holding space inside. The first housing 11 and the second housing 12 are snapped to each other to facilitate the removal and replacement of the device inside the housing 10.

The fan 70 in this embodiment is provided in the mounting cavity 101 formed by the first bracket 50 and the second bracket 60, which can accelerate the outflow of the air in the mounting cavity 101, to further reduce the heat of the air in the mounting cavity 101 to be transmitted to the other electronic devices inside, thereby increasing the overall heat dissipation effect of the hair removal device 100.

In some embodiments, the air inlet 111 and the air outlet 112 are spaced apart on the housing 10, and an area of the air outlet 112 is larger than an area of the air inlet 111.

The air inlet 111 is spaced from the air outlet 112 at a certain distance from each other, which can prevent the hot air coming out of the air outlet 112 from being absorbed again by the air inlet 111, which affects the overall heat dissipation performance of the hair removal device 100. The area of the air outlet 112 is larger than the area of the air inlet 111, which allows the internal airflow not to be blocked in the air duct 20, thus the heat dissipation effect is further improved.

In some embodiments, the air inlet 111 and the air outlet 112 are provided on the same side of the housing 10.

In this embodiment, the air inlet 111 and the air outlet 112 are provided on the first housing 11. The air inlet 111 and the air outlet 112 are provided on the same side of the housing 10, more design space can be left for the other side of the housing 10, and operation components such as the display 80 and the function button 90 can be arranged at will on the other side.

The present embodiment provides a hair removal device 100, and the air inlet 111 and the air outlet 112 are provided on the housing 10. The air inlet 111 is communicated with the air outlet 112 by the air duct 20, so that the air duct 20 is located on two sides of the pulse light source 30, and the airflow generated by the fan 70 enters from the air inlet 111 and flows from two sides of the pulse light source 30, so as to increase the heat dissipation area of the pulse light source 30, which can rapidly take away the heat generated by the pulse light source 30, thereby greatly improving the heat dissipation effect of the hair removal device 100, so that the hair removal device 100 can adopt a higher frequency of the pulse light source 30 to enhance the hair removal efficiency of the device, thereby reducing the user's use time to improve the user's use experience.

Although the present application is described with reference to several embodiments, it should be understood that the terms used are illustrative and exemplary, without limitation. Since the present application can be practiced in a variety of forms without departing from the spirit or substance of the present application, it should be understood that the above embodiments are not limited to any of the foregoing details but should be broadly construed within the spirit and scope defined by the accompanying claims, and therefore all variations and adaptations falling within the scope of the claims or their equivalents should be covered by the accompanying claims.

## Claims

1. A hair removal device, **characterized by** comprising:
a housing, hollow inside, wherein the housing is provided with a light outlet, an air inlet and an air outlet;
a pulse light source, provided inside the housing, wherein pulse light emitted by the pulse light source is emitted from the light outlet;
an air duct, provided inside the housing and in communication with the air inlet and the air outlet, wherein the air duct passes through two sides of the pulse light source, respectively; and
a fan, provided inside the air duct for generating an airflow, to make the airflow enter from the air inlet and pass through the air duct and exit from the air outlet.

2. The hair removal device according to claim 1, wherein the air duct comprises an air inlet duct, a working duct and an air outlet duct communicated sequentially, wherein the working duct comprises a first working duct and a second working duct, the first working duct is located on a side of the pulse light source where the pulse light is emitted from, and the second working duct is located a side of the pulse light source away from the side of the pulse light source where the pulse light is emitted from.

3. The hair removal device according to claim 2, wherein the hair removal device further comprises a reflective cover and a light filter, and an opening at an end of the reflective cover and the hollow inside of the reflective cover form a reflective cavity, wherein the pulse light source is provided in the reflective cavity, and the light filter is provided at an end of the reflective cover from which the pulse light is emitted for sealing the reflective cover.

4. The hair removal device according to claim 3, wherein two sidewalls of the reflective cover are opened with vents opposite to each other, each vent is configured to communicate the air inlet duct with the air outlet duct to form the first working duct for the airflow flowing along the first working duct from a front side of the pulse light source.

5. The hair removal device according to claim 4, wherein the hair removal device further comprises a first bracket, the first bracket is provided with a mounting groove, and an open end of the mounting groove points to a direction where the pulse light is emitted.

6. The hair removal device according to claim 5, wherein the reflective cover is provided in the mounting groove, a gap is provided between an outer wall of the reflective cover and a bottom wall of the mounting groove for communicating the air inlet duct with the air outlet duct to form the second working duct for the airflow flowing along the second working duct from a rear side of the pulse light source.

7. The hair removal device according to claim 6, wherein the hair removal device further comprises a second bracket, the first bracket is provided with a first accommodation cavity and the second bracket is provided with a second accommodation cavity, wherein the second bracket is snapped to the first bracket, to make the first accommodation cavity and the second accommodation cavity combine to form a mounting cavity; and
wherein a side of the first bracket facing the air outlet is opened with a through hole, the through hole is configured to communicate the mounting cavity with the air outlet, a side of the mounting cavity away from the through hole is provided with an opening, and the opening is configured to communicate with the working duct to form the air outlet duct.

8. The hair removal device according to claim 6, wherein the hair removal device further comprises a light gathering assembly, and the light gathering assembly is provided on a side of the light filter away from the pulse light source, wherein a space is formed between the first bracket and the light gathering assembly, and is configured to communicate the air inlet with the working duct to form the air inlet duct.

9. The hair removal device according to claim 8, wherein the light gathering assembly comprises a light gathering frame, a fixing frame and a light outlet cap;
wherein the light outlet cap is connected to the housing, and the light gathering frame is provided within the fixing frame, wherein the light filter, the light gathering frame and the light outlet cap are arranged sequentially along a direction where the pulse light is emitted.

10. The hair removal device according to claim 9, wherein a middle of the light outlet cap is opened with a light outlet for the pulse light to be emitted.

11. The hair removal device according to claim 10, wherein the fixing frame is provided at an inner end of the light outlet cap, the light gathering frame is provided inside the fixing frame, and the light filter is provided at an end of the fixing frame away from the light outlet cap.

12. The hair removal device according to claim 7, wherein the fan is provided inside the mounting cavity.

13. The hair removal device according to claim 1, wherein the air inlet and the air outlet are provided spaced apart on the housing, and an area of the air outlet is larger than an area of the air inlet.

14. The hair removal device according to claim 1, wherein the air inlet and the air outlet are provided on the same side of the housing.

15. The hair removal device according to claim 14, wherein the housing is further provided with a display screen and a function button, and the display screen and the function button are located on a side of the housing away from the air inlet and the air outlet.

16. The hair removal device according to claim 1, wherein the housing comprises a first housing and a second housing, and the first housing is detachably connected to the second housing.
